# EUROPEAN PATENT SPECIFICATION

(11) **EP 4 304 538 B1**
(45) Date of publication and mention of the grant of the patent: **29.07.2026**
(21) Application number: 22767987.5
(22) Date of filing: 10.03.2022
(51) Int. Cl.: A61F 5/56, F04D 19/00, F04D 23/00, A61M 1/08, A61H 9/00, A61H 31/02, A61M 1/00, A61M 16/20

(54) **PRESSURE RELIEF DEVICE AND METHOD FOR OPENING AN AIRWAY**
DRUCKENTLASTUNGSVORRICHTUNG UND VERFAHREN ZUR ATEMWEGSÖFFNUNG
DISPOSITIF DE LIMITATION DE PRESSION ET PROCÉDÉ D'OUVERTURE D'UNE VOIE AÉRIENNE

(30) Priority: 10.03.2021 US 202163159355 P
(43) Date of publication of application: 17.01.2024
(73) Proprietor: Sommetrics, Inc., San Diego, CA 92127 (US); Aerfree, LLC, Woodland Hills, CA 91367 (US)
(72) Inventor: WINTER, David, Vista, California 92081 (US)
(74) Representative: Schiweck Weinzierl Koch Patentanwälte Partnerschaft mbB
(86) International application number: PCT/US2022/019739
(87) International publication number: WO 2022/192530

(56) References cited:
- DE-U1- 202014 101 752
- US-A- 1 739 871
- US-A- 5 839 614
- US-A1- 2005 211 735
- US-A1- 2007 265 585
- US-A1- 2008 163 875
- US-A1- 2008 237 278
- US-A1- 2011 224 633
- US-A1- 2015 126 912
- US-A1- 2020 353 137
- US-B2- 8 540 699

## Description

### BACKGROUND OF THE INVENTION

The following discussion of the background of the invention is merely provided to aid the reader in understanding the invention and is not admitted to describe or constitute prior art to the present invention. Examples of prior art devices are disclosed in US8540699B2 or US2007/265585A1.

The external application of negative pressure to patients for palliative or therapeutic purpose is well established in the medical arts.

U.S. Patents 5,343,878, 7,182,082, and 7,762,263 describe various devices which purport to utilize external application of negative pressure upon the external neck surface of patients. A therapeutic appliance is typically provided that has a surface which is configured to enclose an external area of the throat (the term "throat" as used herein referring to the anterior portion of the neck extending approximately from the chin to the top of the sternum and laterally to a point posterior to the external jugular vein) overlying a portion of the upper respiratory passage. In certain embodiments, these appliances can provide a chamber (e.g., a hollow space filled with air molecules) lying between the interior surface of the chamber and the throat. The therapy appliance is operably connected to an air pump which is configured to produce a partial vacuum in this chamber. Application of a therapeutic level of negative pressure in the chamber elicits movement by pulling the soft tissue in the neck region outwards to maintain an open upper airway and may alleviate conditions such as snoring, sleep apnea, and full or partial airway collapse for example.

Topical negative pressure ("TNP") therapy, sometimes referred to as vacuum assisted closure, negative pressure wound therapy, or reduced pressure wound therapy, is widely recognized as a beneficial mechanism for improving the healing rate of a wound. Such therapy is applicable to a broad range of wounds such as incisional wounds, open wounds and abdominal wounds or the like. TNP therapy assists in the closure and healing of wounds by reducing tissue oedema; encouraging blood flow; stimulating the formation of granulation tissue; removing excess exudates and may reduce bacterial load and thus, infection of the wound. Furthermore, TNP therapy permits less outside disturbance of the wound and promotes more rapid healing. Devices for negative pressure therapy of wounds are known in the state of the art. Thus, for example, WO 1993/009727 A1 describes a device for promoting healing by applying negative pressure on the area of skin that presents the wound and surrounding the wound. The device according to WO 1993/009727 A1 comprises a vacuum device for generating the negative pressure, a hermetic covering of the wound that is functionally linked with the vacuum device, as well as a dressing for positioning the wound within the hermetic covering.

Cupping is a traditional physiotherapy method in traditional Chinese medicine. According to the theory of traditional Chinese medicine, it is related to the fact that people are ill with the related meridians. It is said that "the general rule is not painful, but the pain is not acceptable." Cupping treatment is to introduce the negative pressure formed on the acupuncture point of the human body treatment part by cupping, and to guide the meridian to achieve the medical effect of treating the lesion and regulating the normal function of the human body. Because of the good therapeutic effect of cupping, it has long been respected by people. WO2007128163 discloses cupping therapeutic apparatus" in which vacuum is produced by a vacuum pump.

In these "negative pressure" therapeutic apparatuses and methods there is a possibility that the applied negative pressure could reach values above or below what is required for optimal treatment, induced by body motion that causes variations in chamber volume by compression and or expansion of the chamber, leakage, changes in pressure external to the device and or electrical/mechanical/software malfunction causing a vacuum source to continue operation outside predetermined hysteretic control ranges. This term is defined later in the detailed description of the invention. This is particularly true as the devices are intended for extended use for many hours and under varying conditions during which changes in pressure inside the device may occur; thus, any changes in pressure inside the device must be sensed quickly such that increases or decreases in negative pressure can be made by either active or passive systems to closely maintain the therapeutic level of negative pressure. Further, it is beneficial that when changes in pressure are sensed and corrected, the necessary adjustments are obtained smoothly such that the event(s) are not detected or disruptive to the user. This can be achieved though the design of one or more components of the pressure regulation system to achieve and maintain the hysteretic control ranges.

### BRIEF DESCRIPTION OF THE INVENTION

The invention is defined by the appended claims. The disclosed methods do not form part of the invention as claimed. It is an object of the invention to provide a pressure relief system to support the therapeutic control of the negative pressure within a therapy device comprising a sealed chamber and a seal adapted to form a conforming seal between a device that is intended to attach and seal to a patient's external tissue, such as a face, a neck, an area surrounding a wound, etc. This therapy device is particularly suited for forming a sealed chamber that is configured for the administration of negative pressure to a targeted therapy on the external tissue of an individual without exceeding a maximum vacuum value using active control systems i.e. pressure sensors and vacuum control systems and/or passive-type systems i.e. mechanically actuated pressure relief valve(s). It can also be appreciated that the device could be used in reverse to prevent an excess positive pressure in devices such as a ventilator or resuscitator mask delivering breathing gas to a patient's lungs. The device is specially adapted to work passively in a low pressure environment (9 hPa to 140 hPa pressure) where conventional valve systems tend to be bulky, unreliable and cost prohibitive.

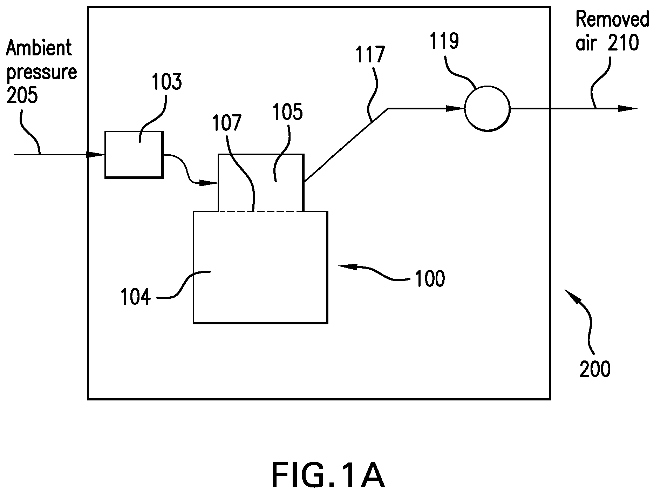

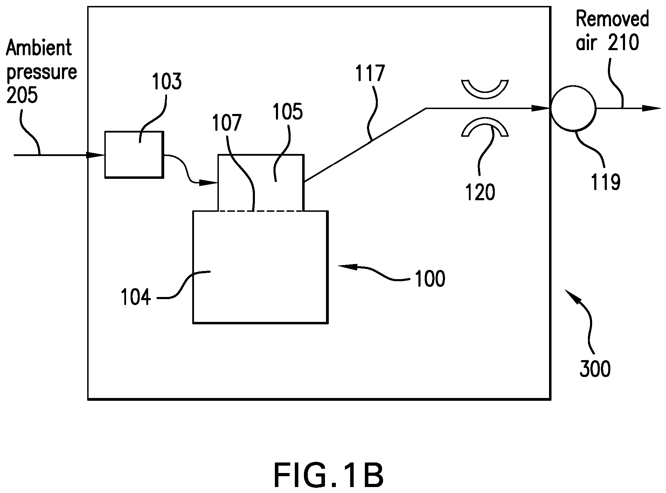

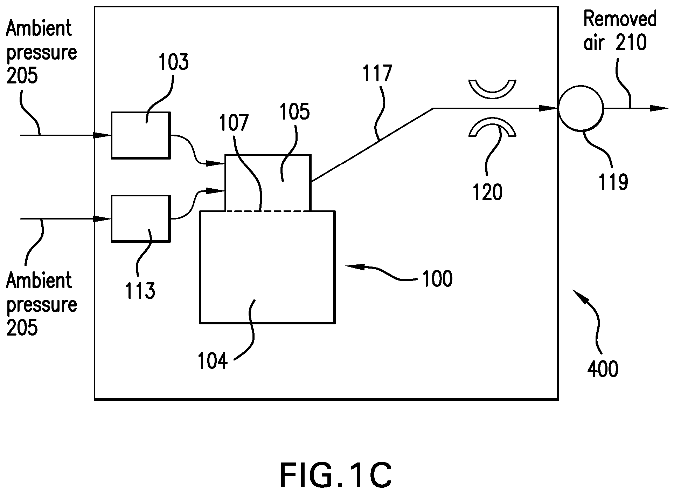

The Invention can be used in at least 3 different negative pressure operating scenarios as illustrated in Figure 1. A first scenario (FIG 1a), is where the negative pressure vacuum source is a vacuum pump operating at or near the therapy negative pressure. In this case, because the vacuum source has a controlled and constrained flow (and so pressure) range, the invention need not utilize a critical orifice to control the gas flow (so as not to exceed the operating capacity of the valve), and the pressure relief valve predominantly can act as a pop-off valve with hysteretic pressure control in instances where the vacuum pump or other events create a level of negative pressure sufficient to open the valve. In the second scenario (FIG 1b), the negative pressure vacuum source is a relatively high-flow vacuum source (hospital vacuum or other exterior vacuum source, for example) that is capable of creating a greater than desired level of vacuum within the vacuum chamber. Such a vacuum source may be unregulated or may be operably connected to a pressure regulator set to a level of negative pressure at, near, or below the target negative therapy pressure. In this second scenario, the devices of the invention may use a critical orifice in conjunction with one or more pressure relief valves to maintain the level of negative pressure in the device, wherein the critical orifice regulates the flow through the device at a maximum level and the pressure relief valve is selected to be in this desired operating range of flow so that the pressure control system again acts as a reliable over-vacuum pressure relief valve. In a third operating scenario (FIG 1c), the negative pressure vacuum source operates at a vacuum level above the target vacuum level in the vacuum chamber. In this scenario, the pressure control system may utilize a flow-controlling critical orifice to control the flow through the system and one or more pressure relief valves that act(s) as a pressure regulator to continuously maintain the operating vacuum near the target pressure level. As described later, a two valve system with or without a critical orifice can be used to provide a controlled "spike" in vacuum when one of the valves is occluded. This may be required, for example, to reopen a collapsed airway. In a similar fashion to these negative pressure scenarios, the pressure control system may be used in positive pressure applications, for example in external artificial ventilation scenarios, to prevent lung injury from an excessive positive pressure.

In related aspects, the present invention relates to methods (not claimed) of applying
negative pressure therapy to an individual in need thereof, comprising mating a therapy device as described herein to the individual, and applying a therapeutic level of negative pressure within the chamber, thereby increasing patency of the airway of the individual. Such methods can be for treatment of sleep apnea; for treatment of snoring; for treatment of full or partial upper airway collapse; for treatment of full or partial upper airway obstruction; for negative pressure treatment of a wound caused by, for example an injury or a surgery; etc.

Thus, in a first aspect, the present invention provides a negative pressure therapy device, comprising:
a chamber element comprising a housing configured to mate with and cover a portion of an individual and thereby enclose an inner chamber volume over the covered portion of the individual, wherein the housing separates the inner chamber volume from an ambient gaseous atmosphere external to the chamber device,
wherein the housing comprises
a vacuum aperture configured to be mated to a vacuum source for evacuation of a gas from within the inner chamber volume formed upon mating of the chamber element with the individual, and
a valve which opens at a first predetermined level of vacuum within the inner chamber volume to create an opening through the housing through which gas from the ambient gaseous atmosphere can enter the inner chamber volume and which closes at a second predetermined level of vacuum within the inner chamber volume, wherein the second predetermined level of vacuum is lower than the first predetermined level of vacuum,
wherein the valve comprises
a semi-elliptical valve head having a marginal edge, an interior side facing the inner chamber volume, an exterior side facing the ambient gaseous atmosphere,
a resilient sleeve at the marginal edge thereof, and
a valve aperture in the valve head which reversibly opens to permit fluid flow therethrough,
wherein the valve head has a generally convex orientation relative to the housing when the valve is in a closed position, and wherein the resilient sleeve is configured and arranged to double over and extend in a rolling manner to apply a torque to the valve head which causes the valve head to invert to a generally concave orientation that assists in opening the valve aperture at or greater than the first predetermined level of vacuum and to revert to its generally convex orientation at or less than the second predetermined level of vacuum; and
wherein the chamber device optionally comprises a compression element that creates a compressed state by applying a compressive force to the exterior side of the valve head when the pressures in the inner chamber volume and the ambient gaseous atmosphere are equal and the valve is closed, wherein the compressive force is configured and arranged to deflect the exterior side of the valve head inward such that the valve aperture is open at the interior side of the valve head but closed at the exterior side of the valve head in the compressed state.

In certain embodiments, the device may further comprise a vacuum source operably connected to the vacuum aperture and configured to evacuate the gas from the inner chamber volume when energized. Such a vacuum source may be a bedside vacuum pump, a vacuum pump provided as an integral component of the housing, "house" vacuum (for example in a hospital or other medical facility where vacuum is available as part of the infrastructure of the facility), etc. Preferably, the vacuum source provides a gas flow rate at the vacuum source that is sufficient to produce a level of vacuum within the inner chamber volume that is greater than the first predetermined level of vacuum when the valve is open. By "level of vacuum" is meant a condition that is below that of the ambient gaseous atmosphere external to the chamber device, typically measured in units of pressure such as the pascal, mm Hg, or mm H₂O. An "increase" in the level of vacuum refers to an increased pressure differential between the ambient gaseous atmosphere and the inner chamber volume. Thus, the term "pressure" refers to this pressure differential, and an increased pressure refers to an increase in this differential.

In certain embodiments, the device comprises a restriction between the inner chamber volume and the vacuum source which produces a critical orifice type of gas flow. In such a device, the gas flow rate through the vacuum aperture is limited by a critical orifice (creating a choked flow condition) positioned between the inner chamber volume and the vacuum source. Preferably, the dimensions of the critical orifice is selected to provide a level of vacuum within the inner chamber volume that is between the first predetermined level of vacuum and the second predetermined level of vacuum when the valve is open. As described hereinafter, the choked flow condition prevents the vacuum source flow rate from being so great as to overwhelm the capacity of the valve to prevent an "over-vacuum" condition within the inner chamber volume; that is, if air is removed by the vacuum source at a greater rate than air can be admitted through the valve, the level of vacuum could rise to a level that is damaging to the tissue that is subject to the vacuum. By restricting the gas flow rate in this manner, the over-vacuum condition is prevented. In various embodiments, the gas flow rate is limited by the "critical orifice" or "choked flow" type of gas flow to a rate of approximately 0.2, 0.5, 1, 2, 3, 4, 5, or 10 liters per minute.

In certain embodiments, the housing further comprises a second valve which opens at a third predetermined level of vacuum within the inner chamber volume to create a second opening through the housing through which gas from the ambient gaseous atmosphere can enter the inner chamber volume, wherein the third predetermined level of vacuum is greater than the first predetermined level of vacuum. As described hereinafter, the device may provide for occlusion of the first valve in order to temporarily create a level of vacuum within the inner chamber volume, where the level of vacuum is controlled by this second valve. Upon removal of the occlusion, the second valve closes and the level of vacuum is again controlled by the first valve.

In various embodiments, the first predetermined level of vacuum and second predetermined level of vacuum are between about 5 hPa and about 75 hPa, more preferably between about 25 hPa and about 55 hPa, and still more preferably between about 31.3 hPa and about 47.1 hPa. As noted above, the third predetermined level of vacuum is greater than the first predetermined level of vacuum. In various embodiments, the third predetermined level of vacuum is between about 47.1 hPa and 137 hPa.

The term "about" as used herein refers to +/- 10% of a given value.

In certain embodiments, the device is a medical device, and most preferably a negative pressure chamber configured to cover an external portion of a human body. Examples of such medical devices include, but are not limited to, negative-pressure wound therapy (NPWT) devices, and continuous negative external pressure (cNEP) therapy devices for maintenance of airway patency by the application of an external vacuum to a human.

In related aspects, the present disclosure provides a method (not claimed) of regulating vacuum using the devices described herein. In certain embodiments the methods are for NPWT or cNEP. By way of example, these methods comprise:
providing a chamber device having a housing that encloses an inner chamber volume comprising a gas to be evacuated from within the chamber device by application of a vacuum, wherein the housing separates the inner chamber volume from an ambient gaseous atmosphere external to the chamber device, wherein the housing comprises a valve which opens at a first predetermined level of vacuum within the inner chamber volume to create an opening through the housing through which gas from the ambient gaseous atmosphere can enter the inner chamber volume and which closes at a second predetermined level of vacuum within the inner volume, wherein the second predetermined level of vacuum is lower than the first predetermined level of vacuum,
wherein the valve comprises
a semi-elliptical valve head having a marginal edge, an interior side facing the inner chamber volume, an exterior side facing the ambient gaseous atmosphere,
a resilient sleeve at the marginal edge thereof, and
a valve aperture in the valve head which reversibly opens to permit fluid flow therethrough,
wherein the valve head has a generally convex orientation relative to the housing when the valve is in a closed position, and wherein the resilient sleeve is configured and arranged to double over and extend in a rolling manner to apply a torque to the valve head which causes the valve head to invert to a generally concave orientation that assists in opening the valve aperture at or greater than the first predetermined level of vacuum and to revert to its generally convex orientation at or less than the second predetermined level of vacuum, and
wherein the chamber device optionally comprises a compression element that creates a compressed state by applying a compressive force to the exterior side of the valve head when the pressures in the inner chamber volume and the ambient gaseous atmosphere are equal and the valve is closed, wherein the compressive force is configured and arranged to deflect the exterior side of the, wherein the compressive force is configured and arranged to deflect the exterior side of the valve head inward such that the valve aperture is open at the interior side of the valve head but closed at the exterior side of the valve head in the compressed state;
operably connecting the chamber device to a vacuum source to evacuate the gas from the inner volume, wherein opening and closing of the valve regulates the level of vacuum within the inner volume to a range between the first predetermined level of vacuum and the second predetermined level of vacuum.

### BRIEF DESCRIPTION OF THE DRAWINGS

FIG. 1a is a schematic representation of an embodiment of the invention showing exemplary a therapy device 200. Air pump 119 removes air along a pathway 117 from a vacuum chamber 100 portion of the device comprising a main chamber 104 and a protected volume 105 separated by a physical structure 107. This air 210 is exhausted through the pump and into the external atmosphere. A pressure relief valve 103 opens at a first predetermined level of vacuum within vacuum chamber 100 to admit air into vacuum chamber 100 and thereby prevent an overpressure condition from occurring; and closes at a second, lower, predetermined level of vacuum such that the desired level of vacuum within vacuum chamber 100 is maintained within a desired range..
FIG. 1b is a schematic representation of a second embodiment of the invention showing exemplary a therapy device 300. Air pump 119 removes air along a pathway 117 from a vacuum chamber 100 portion of the device comprising a main chamber 104 and a protected volume 105 separated by a physical structure 107. This air 210 is exhausted through the pump and into the external atmosphere. A pressure relief valve 103 opens at a first predetermined level of vacuum within vacuum chamber 100 to admit air into vacuum chamber 100 and thereby prevent an overpressure condition from occurring; and closes at a second, lower, predetermined level of vacuum such that the desired level of vacuum within vacuum chamber 100 is maintained within a desired range. A critical orifice 120 is placed in-line with the air pump 119 to limit the rate of airflow out of vacuum chamber 100 so that inward airflow through pressure relief valve 103 is not overwhelmed by removed airflow created by air pump 119.
FIG. 1c is a schematic representation of a third embodiment of the invention showing exemplary a therapy device 400. Air pump 119 removes air along a pathway 117 from a vacuum chamber 100 portion of the device comprising a main chamber 104 and a protected volume 105 separated by a physical structure 107. This air 210 is exhausted through the pump and into the external atmosphere. A pressure relief valve 103 opens at a first predetermined level of vacuum within vacuum chamber 100 to admit air into vacuum chamber 100 and thereby prevent an overpressure condition from occurring; and closes at a second, lower, predetermined level of vacuum such that the desired level of vacuum within vacuum chamber 100 is maintained within a desired range. A critical orifice 120 is placed in-line with the air pump 119 to limit the rate of airflow out of vacuum chamber 100 so that inward airflow through pressure relief valve 103 is not overwhelmed by removed airflow created by air pump 119. A second pressure relief valve 113 which opens at a third predetermined level of vacuum within vacuum chamber 100 that is different from the first predetermined level of vacuum and that closes at a fourth, lower, predetermined level of vacuum is provided to provide an ability to temporarily alter the vacuum level in vacuum chamber 100 by selecting which pressure relief valve is permitted to operate.

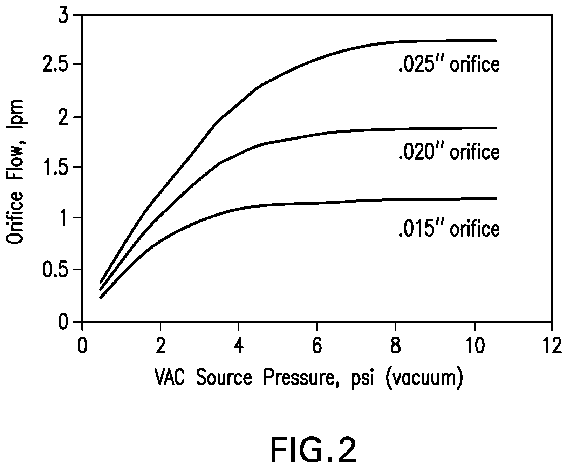

FIG. 2 is a graphical representation of air flow through critical orifices with different diameters as a function of vacuum source pressure.

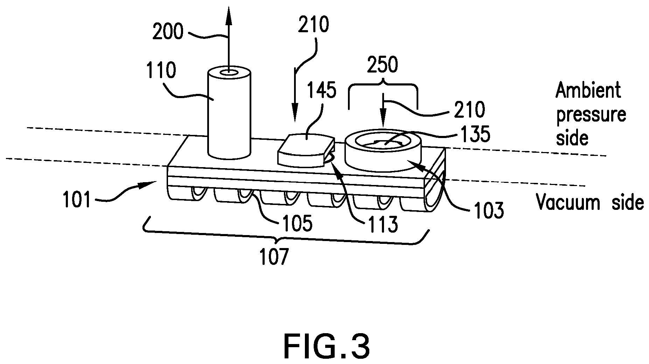

FIG. 3 is an illustrative drawing showing the an exemplary vacuum control element 101 of the invention. A physical structure 107, depicted as a series of discontinuous air passages, create a space 105, referred to herein as a "protected volume" which keeps a skin surface or other material from occluding the undersides of vacuum source connector 110 and pressure relief valves 103 and 113. In this drawing, the leaflets of pressure relief valves 103 and 113 are not shown, as these are obscured by the walls of aperture structure 250 and compression element 145, respectively. The direction of flow of air outward from the vacuum side of the vacuum control element through the vacuum source connector 200 is shown by an arrow, as are the inward direction of flow of air into the negative pressure therapy from the ambient side of the device 203 into the vacuum side of the device 101 when one or more of the pressure relief valves are opened. The dashed lines represent the delineation between the ambient side of the device and the vacuum side of the device. Each of the first pressure relief valve element 103 and second pressure relief valve 113are slightly compressed by compression elements 135 and 145, respectively.

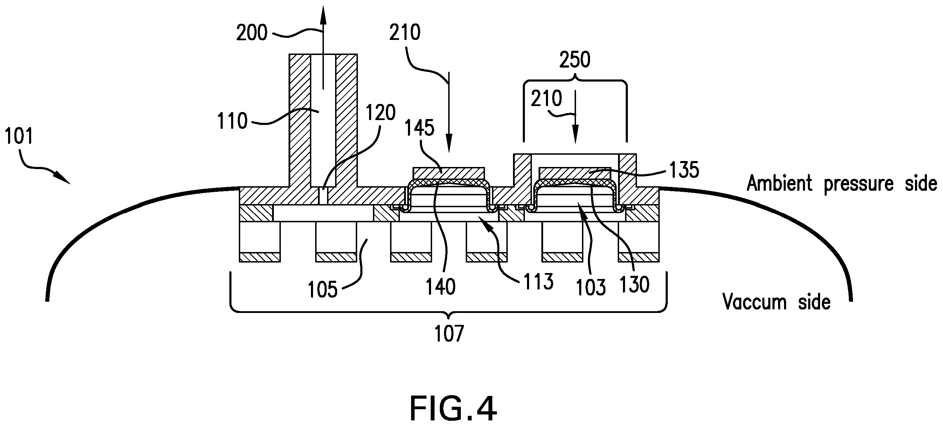

FIG. 4 is an illustrative drawing of a cross-section of a an exemplary vacuum control element 101 of the invention as shown in Fig. 3. Pressure relief valves 103 and 113 each comprise a dome-shaped semi-elliptical valve element 130 and 140, respectively, the dome of which are each slightly compressed by compression structures 135 and 145.

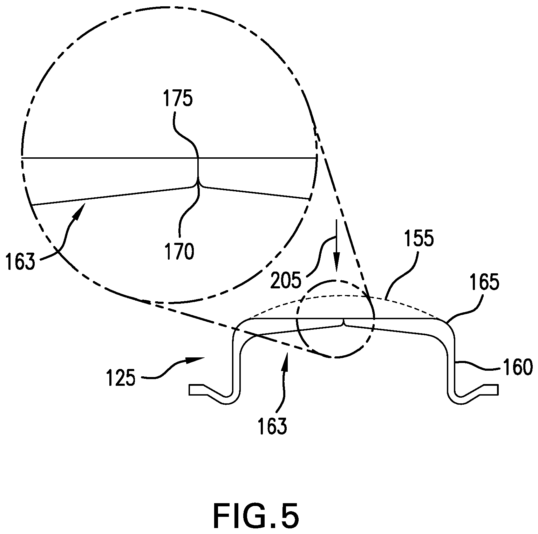

FIG. 5 is an illustrative drawing of a cross section of a dome-shaped semi-elliptical valve element 125 equivalent to elements 130 and 140 in Fig. 4. Each is shown in its compressed state, with a dashed line 155 illustrating the non-compressed state of the valve head. Semi-elliptical valve element 125 comprises a resilient sleeve 160 at the marginal edge 165 of the valve head. Because of the compression, the leaflets forming the opening, or valve aperture, of the valve head 163 become slightly opened at the bottom surface, although remaining sealed at the tope surface. This is also shownin the enlarged view for emphasis. The direction of air flow from the ambient side of the valve into the negative pressure device chamber when the semi-elliptical press relief valves are opened 210 is shown with an arrow.

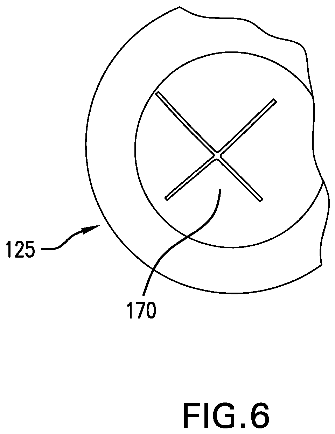

FIG. 6 is a photograph of semi-elliptical valve head 125 in its compressed (or "pre-loaded") state showing the interior surface of the valve aperture 170 where the valve leafletsare partially open toward the interior side of the valve head but remain closed at the exterior side of the valve head such that the valve is sealed to airflow.

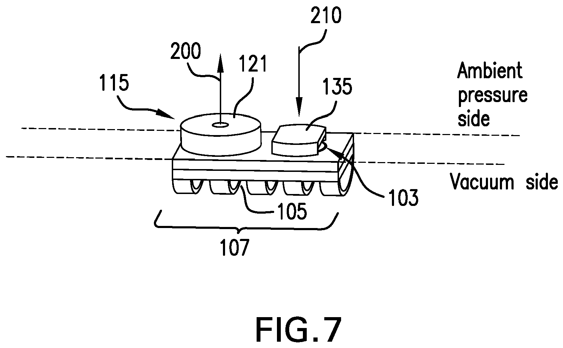

FIG. 7 is an illustrative drawing of a second exemplary vacuum control element 115 of the invention showing a protected minimum volume 105 protected from occlusion by physical structures 107, an integral vacuum pump 121, direction of flow of air through the vacuum pump housing 200, direction of flow of air 210 into the negative pressure therapy device chamber through pressure relief valve 103 , and a compression element 135 for relief valve 103.

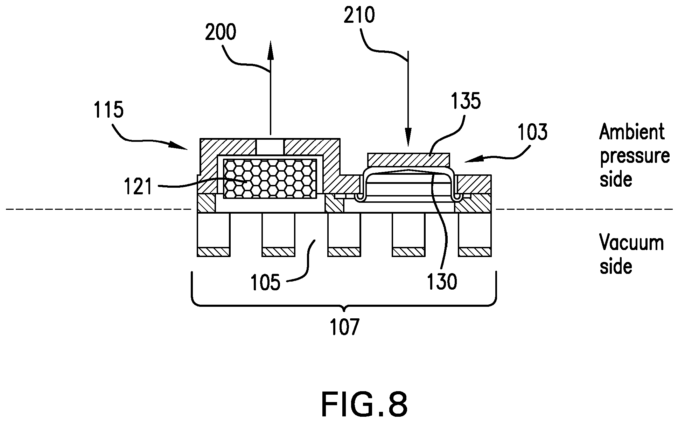

FIG. 8 is an illustrative drawing of a cross-section of the pressure relief valve device 115 depicted in Fig. 7, showing a first pressure relief valve element 103, protected minimum volume 105 physical structures 107, an integral vacuum pump 121, direction of flow of air out of the negative pressure therapy device 200, direction of air flow 210when the pressure relief valve 103 is opened, a first semi-elliptical valve head 130 and a first compression element 135.

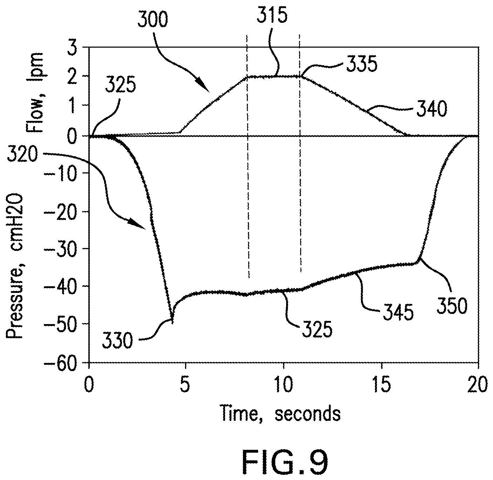

FIG. 9 is a graphical representation of a 20 second pressure relief flow cycle depicting flow through a pressure relief valve (in liters per minute) as a function of time (upper trace 300) and corresponding vacuum level (in cm H₂O) (lower trace 320). As the vacuum increases beginning at 325, flow through the pressure relief valve is essentially 0. After the "cracking pressure" 330 is reached, the pressure relief valve opens. For a period of time, the flow through the pressure relief valve is approximately constant 315 and the change in the level of negative pressure in the device remains approximately flat 325. When the pressure relief valve leaflets begin to close, a corresponding reduction of flow through the pressure relief valve begins at 335, resulting in a decay in flow 340 until valve closure at350.

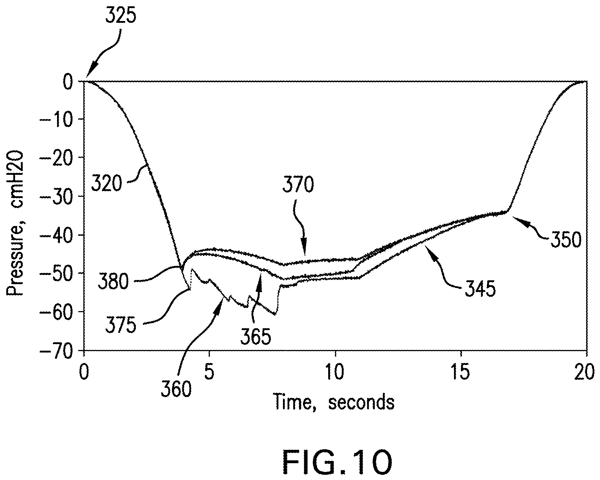

FIG. 10 is a graphical representation of vacuum level (in cm H₂O) as a function of time for a dome-shaped semi-elliptical valve element that lacks the compression depicted in Fig. 6 during three flow cycles of the same valve element. The lower trace 360 is the first cycle of the valve, and shows that as the vacuum level begins to increase at 325, a constant increase in vacuum 320 occurs until a "cracking" pressure 375 (that is, valve opening) is achieved at about 57 cm H₂O and the valve exhibits a somewhat nonlinear pressure relief profile until a stable flow of air through the valve is achieved at about 7.5 minutes. If the valve is then allowed to return to its fully closed position for a short time, a second flow cycle results in pressure traces 365 and 370. These subsequent flow cycles exhibit a lower, and reproducible cracking level of vaccum 380 and an earlier stable air flow, which results in a steady loss of vacuum level 345 until the pressure relief valve element leaflets begin to close 350.

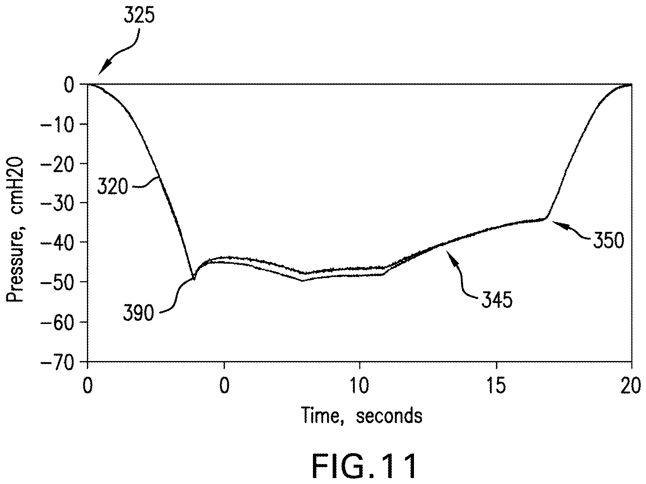

FIG. 11 is a graphical representation of vacuum level (in cm H₂O) as a function of time for a dome-shaped semi-elliptical valve element that is operated with the compression element 135 depicted in Fig. 7 during multiple flow cycles of the same valve element. as the vacuum level begins to increase at 325, a constant increase in vacuum 320 occurs until a "cracking" pressure 390 (that is, valve opening) is achieved at about 50 cm H₂O and the valve exhibits a reproducible pressure relief profile throughout the period of use. The valve pre-compression substantially eliminates the variation in initial cracking pressures seen in FIG. 10.

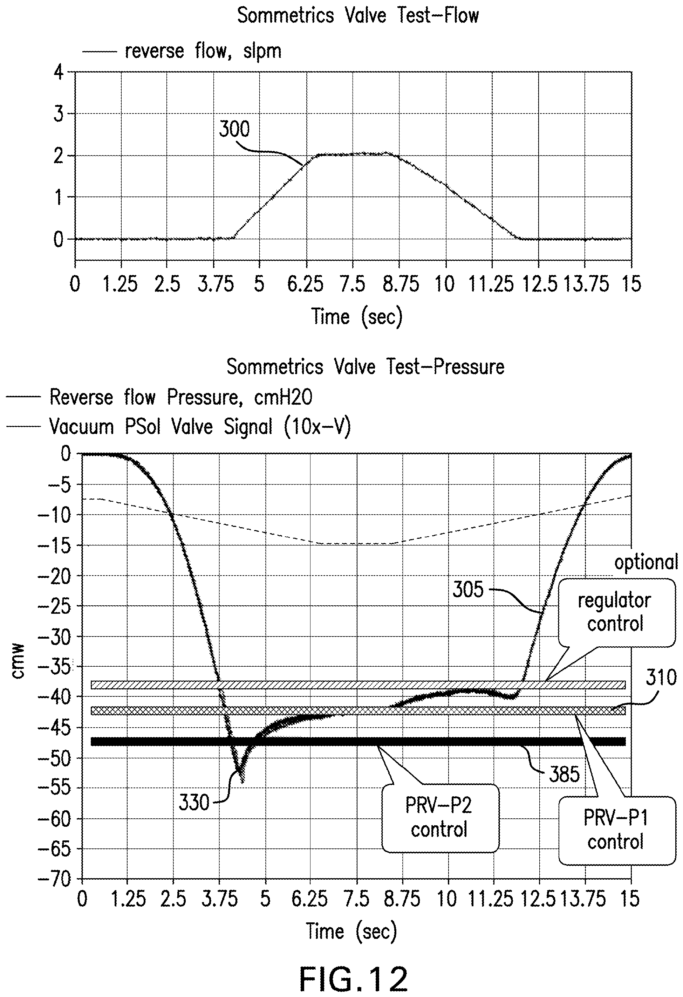

FIG. 12 is a graphical representation of air flow through a pressure relief valve device comprising two pre-compressed dome-shaped semi-elliptical valve elements. The flow cycle graph contains several overlapping traces with the upper trace 300 showing the flow through the pressure relief valve device in liters per minute as a function of time and the lower trace 305 showing the resulting changes in vacuum level in cm H₂O as a function of time. Cracking pressure of the first valve 330 occurs at about four seconds. The first valve element maintains a stable vacuum level 310 at about 42 cm H₂O, the second valve element maintains a stable vacuum level 385 at about 47 cm H₂O, and a critical orifice maintains a regulated control at about 37 cm H₂O.

### DETAILED DESCRIPTION OF THE INVENTION

The present invention, and the various features and advantageous details thereof, are explained more fully with reference to the non-limiting embodiments that are illustrated in the accompanying drawings and detailed in the following description. It should be noted that the features illustrated in the drawings are not necessarily drawn to scale. Descriptions of well-known components and processing techniques are omitted so as to not unnecessarily obscure the present invention. The examples used herein are intended merely to facilitate an understanding of ways in which the invention may be practiced and to further enable those of skill in the art to practice the invention. Accordingly, the examples should not be construed as limiting the scope of the invention. In the drawings, like reference numerals designate corresponding parts throughout the several views.

In negative pressure therapeutic apparatuses and methods, there is a potential for the negative pressure to reach vacuum levels above those required for optimal treatment, or even that are dangerous to living tissue exposed to such vacuum levels. These undesired values may be induced by the vacuum source itself being an unregulated and high-flow source; electrical/mechanical/software malfunction either at an external flow regulator or at the vacuum source itself, resulting in a vacuum flow sufficient to create an excessive vacuum level; by body motion that causes variations in chamber volume due to compression and or expansion of the chamber and or movement of tissue into the chamber upon application of negative pressure; leakage that is in excess of any designed airflow (for venting or cooling for example) and or that is due to momentary seal disruption; changes in pressure due to temperature change; changes in pressure external to the device, etc.

In devices that are intended for extended use for many hours while the user is partially sedated, sedated and or asleep under varying conditions during which changes in pressure inside the device may occur, the device itself must be capable of responding to such changes in vacuum to limit pressure changes for the safety of the user. Thus, any changes that would result in pressure changes inside the device must be sensed quickly and responded to by a vacuum source, a control system and or one or features of the device such that increases or decreases in negative pressure can be made to closely maintain the therapeutic level of negative pressure.

It is an object of the invention to provide a pressure regulating and/or relief device as a component of a negative pressure apparatus. In various embodiments, the device is configured to establish a desired negative pressure, and to maintain the desired vacuum level within the vacuum chamber of the apparatus. In other embodiments, the device is configured to relieve undesired excess vacuum. In particular, a valve is provided that opens when a first negative pressure is reached and reseal when a second (lower) negative pressure reached, thereby providing for maintenance of a desired therapeutic pressure range within the vacuum chamber of a negative pressure device. In various aspects described below, the pressure regulating/relief device can be in the form of one or more pressure relief valve element(s) that are integral to the negative pressure therapeutic apparatus. The valve element(s) are configured to open to allow external air to flow into the vacuum chamber of the apparatus when the upper bound of a desired vacuum range is reached, thereby preventing the vacuum source from creating an over-pressure situation; and close when the lower bound of a desired vacuum range is reached, thereby prevent an under-pressure situation. Thus, the valve element(s) constrain the vacuum level within the vacuum chamber to a desired range.

As used herein, a pressure relief valve element(s) is defined as a component(s) of a vacuum device that is used to control pressure in the device when one or more predetermined levels of negative pressure is exceeded. In certain embodiments, the opening and closing of the pressure relief valve element(s) provide for hysteretic control, such that the level of vacuum is controlled by flow from the ambient side of the device into the vacuum chamber in a range, most preferably to maintain the level of vacuum to a range that is approximately within the mid-range of opening and closing values of the pressure relief valve(s).

In certain embodiments, the devices of the present invention may be used with a vacuum source that, if not regulated properly, could exceed the ability of the valve element(s) to compensate; that is, the vacuum source removes air from the vacuum chamber faster than the valve element(s) admit air. As used herein, an "elevated vacuum source" refers to such a source of negative pressure that has an air flow rate greater than can be accommodated by one or more pressure relief valves elements. To protect against such an elevated vacuum source, the devices of the present invention may optionally comprise a restriction known as a "critical orifice" in the flow path between the vacuum chamber and the vacuum source. The critical orifice converts variable flow into a controlled flow downstream to aid in achieving the desired levels of negative pressure in the therapy device and approximately conform to the operational range(s) of one or more pressure relief valve element(s).

A schematic description of embodiments of the pressure control system(s) can be seen in FIG 1a, FIG 1b and FIG 1c each illustrating three possible negative pressure regulating scenarios. In an embodiment of the invention a first scenario, as shown in FIG 1a., device 200 comprises a system pump 119, which preferably comprises associated processors, sensors and pre-programmed parameters. The system pump 119 is operably connected to a vacuum chamber 100 along a flow path 117. vacuum chamber 100 comprises a minimum volume 105, and a chamber volume 104. Each of these are operably connected to a first pressure relief valve element 103. The minimum volume 105 is fluidly connected to chamber volume 104 such that the system pump 119 evacuates air from both volumes under normal operation. A barrier 107 separates the minimum volume 105 from chamber volume 104 in the sense that it prevents objects (e.g. a skin surface of a patient being treated using negative pressure) from occluding the vacuum pump and pressure relief valve 103. This provides a discrete area in which the air flow (i.e. vacuum source) and pressure relief valve(s) can create a loop of airflow independent of the chamber volume, preventing the skin surface from being damaged by an excessive pressure condition. Thus, in this preferred embodiment, continued application of vacuum is avoided in the event of an inadvertent blockage of the minimum volume and pressure relief valve system.

During normal operation, the pressure relief valve element 103 can serve as a "pop-off" type valve, operating in response to any changes in pressure within the chamber element, thus providing a safety element in the unlikely event of pump "run-away". During operation of the device, system pump 119 creates a flow of air moving through the device, the minimum volume 105 and chamber volume 100 and out of the device 210, until a desired level of negative pressure is obtained in the device. In the event the negative pressure exceeds the selected pressure relief valve tolerances, the pressure relief valve element 103 opens, allowing airflow from the ambient air outside the pressure relief valve into the minimum volume 105 and chamber volume 104 until the vacuum pressure decreases to a level where the pressure relief valve element 103 closes.

In Fig. 1a, the system pump is depicted as an element of the device 200. This is for illustration purposes only. The system pump may be a vacuum source that is separate from the device itself, for example being a bedside or tabletop unit, or house vacuum in a medical facility, without altering the function of the device. The system pump may be an unregulated vacuum source, or may be regulated, either within the system pump itself or by providing a vacuum regulator that is separate from the system pump.

In a further embodiment of the invention as schematically depicted in FIG. 1b, a second scenario entails the flow originating from a vacuum source 119 that is separate from the device 300 (for example a hospital suction vacuum source or vacuum pump). In this embodiment, a critical orifice element 120 is used to limit the ability of the vacuum source to overwhelm the ability of pressure relief valve 103 to compensate for an over-pressure situation. The vacuum flow out of the device 300 is regulated by an aperture within the critical orifice element that is selected to provide an approximately desired level of airflow from the vacuum source to achieve a controlled level of negative pressure in the device. In preferred embodiments, this desired level of airflow is at approximately the midpoint of airflow into the device that pressure relief valve 103 can supply.

By way of example, a dome-shaped semi-elliptical valve element as described herein may open at a first level of vacuum, but close at a second, lower, level of vacuum (a "hysteresis"). Because of valve leaflet movement ("flutter"), this hysteresis creates a range in the airflow through the valve as the valve is open for airflow. This range of airflow can create a managed, hysteretic range of vacuum levels with vacuum chamber 100. Using a critical orifice type of airflow out of the device on the vacuum side to create an airflow that is within this hysteretic range provides an additional level of regulation resulting in a stable vacuum level.

In a further embodiment of the invention as schematically depicted in FIG. 1c, a third scenario entails the flow originating from an elevated exterior vacuum source 119 operating at a vacuum flow capacity above the desired flow rate through a critical orifice 120 operably connected to the minimum volume 105, chamber volume 104 and one or more pressure relief valves (103, 113). In this scenario, a critical orifice 120 controls the flow through the device to create the desired level of negative pressure within the device. In this embodiment the first pressure relief valve 103 opens at a first vacuum level to allow gas flow into the device such that a first level of negative pressure can be maintained, while the second pressure relief valve 113 opens at a different vacuum level to allow gas flow into the device such that a different level of negative pressure can be maintained. In various embodiments, the choice between the first and second pressure relief valves can be controlled manually or can be controlled electronically by a controller in device 400. In certain embodiments, the pressure relief valve (103 or 113) that opens at the lower vacuum level will operate as the default valve, and the level of negative pressure provided by that pressure relief valve will provide the working negative pressure level. By manually or electronically forcing closure of the default valve, the second pressure relief valve that opens at a higher vacuum level comes into play, and the level of negative pressure provided by the second pressure relief valve becomes operable. Once the forced closure is relieved and the default valve opens, the working negative pressure level is resumed.

The vacuum level regulating and relief device may further be configured to apply different types of therapy target vacuum level during use due to body movement, position of the device/user or the onset or alleviation of upper airway narrowing or obstruction. This approximately constant target negative vacuum level may have a predetermined range, a target vacuum level with upper and lower limits, i.e. target vacuum level range, that comprises a maximum value, a minimum value and a midpoint value wherein the maximum and minimum values are each within about 5 hPa, and more preferably within about 2 hPa of the midpoint value (+/- ~5 hPa, and preferably ~2 hPa) wherein the midpoint value is between about 10 hPa and about 60 hPa. The target vacuum level range may be further noted to accommodate for material and manufacturing variations in the vacuum level relief valves that may range up to about less than 25%, less than about 20%, less than about 10% and more preferably less than about 5% or lower of target values.

In an embodiment of the invention that utilizes an elevated vacuum source and a critical orifice, negative pressure within the chamber element is achieved though the combined use of the flow rate provided by the dimensions of the critical orifice and the designed pressure range of one or more of the pressure valve element(s). By way of example, an elevated vacuum source may have an operating flow capacity greater than 5 liters per minute, and the critical orifice is selected to regulate the flow of air out of the chamber element to approximately 2 liters per minute. In an embodiment of the pressure regulating and relief device wherein an elevated vacuum source is utilized and operably connected to a critical orifice the diameter of the critical orifice is approximately 0.6 millimeters with a length of approximately 1.3 millimeters, providing a flow rate of approximately 2 liters per minute and one (or more) pressure valve element(s) opens and closes to provide an approximate negative pressure in the vacuum chamber of about between 31 hPa and about 48 hPa.

A graphical representation of how selection of critical orifice(s) results in a limit on achievable vacuum levels is shown in FIG 2. Operating characteristics of the critical orifice can be seen as the flow through the critical orifice versus the vacuum source pressure. As vacuum pressure increases, the flow through each critical orifice increases until the maximum flow through the critical orifice is achieved. This is referred to as a choked flow condition. As seen in FIG. 2, as vacuum pressure increases from 0 psi to approximately 7 psi, each critical orifice (0.015 inches, 0.020 inches and 0.025 inches in diameter) reach their maximum flow (approximately 1.0 lpm, 1.5 lpm and 2.5 lpm respectively), after which any further increase in vacuum pressure does not increase the flow through the critical orifice. A critical orifice may be as simple as a hole in a surface, often referred to as an orifice plate, or may take the form of an elongated tube, in each case providing a flow path which restricts flow to a rate that is smaller than that which would occur if the flow path was not restricted in this manner. Methods for determining the dimensions of a critical orifice required to achieve a desired choked flow rate are well known in the art.

While described herein with regard to a device configured to provide a negative pressure environment, t is to be understood the invention could be used in an analogous way with a positive pressure source, such as a mechanical ventilator or resuscitator, which may be operating at target gas flow rates up to, for example, 100 liters per minute.

In an embodiment of the invention, a regulated vacuum source may be configured as integral to the device and to provide an approximately constant target negative pressure within the chamber element when the therapy device is mated to the individual and a therapeutic level of negative pressure is applied within the chamber element. By "approximately constant" as used herein is meant that the negative pressure is maintained within a predetermined range during normal intended use without perturbations resulting from short-term transient spikes or drops (increases or decreases) in negative pressure from movement, swallowing, sneezing etc. In preferred embodiments, "approximately constant" is within +/- 25%, more preferably within +/-20%, still more preferably within +/- 10%, and most preferably within +/- 5%, of a design value during use of the device. As described hereinafter, the pressure control system of the regulated vacuum source is also preferably configured to accommodate pressure changes from unintended leakage into the vacuum chamber by rapidly increasing pump airflow when the characteristics of a pressure drop are indicative of a loss of seal integrity, followed by a return to normal airflow when the operating vacuum level is regained.

The term "pressure relief components" refers to an element designed to only open when a first (e.g., excessive or undesired) negative pressure threshold is exceeded and to close when a second (lower or desired) level is re-achieved (a "pop-off valve" type of use); or as a dynamic element that allows a metered flow of air in one direction and is designed to open and close to maintain a desired hysteretic control pressure range (a "regulator" type of use). By "hysteretic control" is meant a control system in which the output reacts less rapidly than it otherwise would by taking recent system history into account. For example, a thermostat controlling a heater may switch the heater on when the temperature drops below A, but not turn it off until the temperature rises above B. (For instance, if one wishes to maintain a temperature of 20 °C then one might set the thermostat to turn the heater on when the temperature drops to below 18 °C and off when the temperature exceeds 22 °C). In the present case, if one wishes to maintain a vacuum level of 25 hPa, a pressure valve may open when the vacuum level reaches 27 hPa, but close when the pressure reaches 23 hPa.

In certain embodiments, the pressure relief components can be in the form of a discrete pressure relief valve element or a pressure relief valve element integrated alongside an air pump element, control element or any aspect of the negative pressure device. In preferred embodiments the pressure relief valve element is contained within a protective housing, i.e a minimum volume to avoid inadvertent blockage. The minimum volume is created via a physical structure that isolates a portion of the vacuum side of the device such that if the physical structure becomes blocked, by ingress of tissue for example, the minimum volume is maintained and directs flow of vacuum though the pressure relief valves.

In certain embodiments the pressure relief components may comprise one or more check valves, diaphragm valves, pinch valves, pop-off valves, solenoid valves, slit valves or any suitable valve for the control and regulation of pressure within a negative pressure device. The pressure relief valve device being designed to be functionally operable through all ranges of vacuum with a preferred vacuum pressure of about between 10 hPa and about 140 hPa, regardless of applied vacuum above these levels.

In certain embodiments, as seen in FIG 1c, FIG 3 and FIG 4, the pressure relief components may comprise multiple pressure relief valves with for example a first pressure relief valve 103 having an initial operating vacuum value and at least a subsequent second pressure relief valve 113 having an operating vacuum value that is the same or higher that the first pressure relief valve 103 and so on, thereby providing redundancy in the event the first pressure relief valve 103 is inadvertently occluded or for providing a different operating vacuum value when the first pressure relief valve 103 is manually occluded. In a further embodiment the first pressure relief valve 103 comprises a sealable aperture 250 that can be physically occluded, for example with a finger, thereby engaging the second pressure relief valve 113 and second pressure relief valve operating pressure value(s) enabling the higher vacuum range of the second pressure relief valve 113 to be achieved. A higher level of negative pressure within a negative pressure device can be used to restore the patency of a collapsed upper airway, for example.

While described herein for use in negative pressure applications, in an analogous way the invention could be used in positive pressure applications such as mechanical patient ventilation to avoid over-pressure situation that might damage an airway in a patient.

In certain embodiments the pressure relief valve element is a one-way valve for the releasing of excess negative pressure within a negative pressure device comprising an elastomeric slit valve with an interior surface that is disposed to face the interior of the negative pressure device and an exterior surface disposed to face the exterior of the device.

In certain embodiments the pressure relief valve is a "slit valve" comprising one or more slits located on the central axis, extending perpendicular to the central axis and creating an aperture when the slit valve is opened and re-sealing when the slit valve is closed. In certain embodiments the slit valve is made of a deformable, elastomeric material that may be comprised of any type elastomeric material including but not limited to linear low density polyethylene (LLDPE), thermoplastic elastomer (TPE), polyurethane (PUR) and EPDM or rubbers (for example silicone rubber).

In certain embodiments as seen in FIG 5, the pressure relief valve is a dome-shaped semi-elliptical valve element comprising a semi-elliptical valve head 125 having a marginal edge 165, an interior side facing 163 the low pressure side of the vacuum device, an exterior side facing the ambient gaseous atmosphere, a resilient sleeve 160 at the marginal edge and a valve aperture 175 which reversibly opens to permit flow therethrough. As used herein a "semi-elliptical valve head" is defined as a valve in the form of a flexible material in the shape of a plane curve comprising a partly compressed convex surface initially facing toward the exterior of a chamber volume 100 and a concave surface facing toward the low pressure side of the device such that, when the valve is opened, the fluid flow 205 proceeds from the ambient pressure side of the device toward the low pressure side. As used herein a "resilient sleeve" at the marginal edge of the semi-elliptical valve head is defined as the flexible sidewalls of the semi-elliptical valve head that are configured and arranged to double over and extend in a rolling manner to apply torque to the valve head which causes it to invert to a generally concave orientation that assists in opening the valve aperture(s) on the apex of valve head as vacuum within the chamber element reaches a first pre-determined level of vacuum. Examples of suitable valves, processes to make them and so on may be found, for example, in US Patents 1,739,871, 2,147 164, 5,115,950, 5,839,614, 5,927,566, 6,273,305, 6,405,901, and 7,784,652.

In preferred embodiments a semi-elliptical valve head is made from silicone rubber, wherein the valve aperture contains one or more slits (Fig. 6, 170) at the apex of the semi-elliptical surface, thereby forming the leaflets of the valve. When a vacuum is applied and a predetermined level of vacuum within the vacuum chamber is exceeded, the resilient sleeve travels inward toward the interior of the vacuum chamber opening the leaflets of the valve, thereby allowing air to pass though and relieving the vacuum within the interior of the chamber. The leaflets of the valve head would remain open until the negative pressure within the chamber reaches a desired range, relaxing the leaflets of the valve head, re-sealing the leaflets, and returning the resilient sleeve of the semi-elliptical valve head to its original state.

In preferred embodiments the valve leaflets have a "cracking" or "opening" level of vacuum pressure that is substantially consistent throughout every usage. As used herein, "cracking pressure" is defined as the level of pressure required to open the leaflets of the valve allowing air flow. As used herein, a substantially consistent cracking pressure is defined as a variation in repeated openings that is preferably less than about 25%, less than about 20%, less than about 15%, less than about 10% and most preferably less than about 5%, regardless of how long the leaflets have been maintained in a closed position.

A graphical representation of an embodiment of the device operation can be seen in FIG. 9 which shows a pressure relief valve operation during an exemplary 20 second flow cycle. It is noteworthy that valve operation can and may occur in shorter or longer time period as needed. The upper trace 300, shows the flow in liters per minute as a function of time and the lower trace 320 shows the corresponding pressure as a function of time following a vacuum perturbation in excess of the desired vacuum level. Flow 325 gradually increases to a rate where the critical orifice takes over to provide a constant flow 315. During the same time, one can see the pressure drop until it reaches the cracking pressure 330 of a first pressure relief valve element (FIG. 3, 4, 7, 8; 103). The pressure relief valve element opens allowing the excess negative pressure to be released until a balance between the flow and the pressure is achieved as seen in the flow trace 315 and corresponding pressure trace 325. The pressure relief valve operation may be dynamic in that it may open fully, partially or open and close during operation to stabilize the pressure within the device to predetermined values. In the operation shown in FIG. 9, there is a decay in flow 340 and corresponding increase in pressure 345 is observed until the pressure relief valve closes at 350.

Silicone rubber is highly resistant to methods of sterilization used for the preparation and processing of medical and food processing equipment, for example, including but not limited to temperature, chemical and radiation. A known property of silicone rubber, however, is its ability to "self-heal," either partially or substantially, regaining much or all of its original mechanical properties and shape over time after being distorted. Surfaces that regain contact with one another have shown the ability to re-adhere when opposing faces of separated surfaces are brought into contact with one another. The longer the surfaces remain in contact with one another the greater the adhesion is observed. On medical devices, a risk resulting from this healing property is that there is increased resistance to a first perturbation or distortion leading to an impeded and/or decreased precision of initial liquid or fluid flow or delivery (i.e. cracking pressure for valve-type applications). Stated differently, if leaflets of a silicone rubber valve are left in contact one another in a closed position, the initial vacuum pressure required to open the leaflets can increase relative to second, third, etc. openings that follow shortly thereafter. This presents a challenge for manufacturers who often have to add a non-stick surface treatment to facilitate separation of the leaflet surfaces or provide special handling and storage instructions for valve opening. These pre-treatments aid but do not fully eliminate the self-healing properties of the silicone rubber. This also not only increases the cost to manufacturers but also the risk of contamination of the products.

In order to standardize the cracking pressure required to open the pressure relief valve and avoid inconsistencies of the initial cracking pressure where the valves are made of materials that contain self-healing adhesion characteristics such as with silicone rubber, cracking pressure variation can be further eliminated, in addition to preconditioning, by means of mechanical design. In an embodiment of the device shown in Fig. 4, the pressure relief valve element(s) (103, 113) comprises a compression element (135, 145) which applies a compressive force to the exterior side of the valve head in the closed state (that is, when the pressures on the vacuum side and the ambient gaseous atmosphere are equal). As used herein a compression element is defined as a structural element that applies a compressive force to the convex surface of the semi-elliptical valve head. A compressive force is defined as a physical force pressing on an object so as to make it more compact. In embodiments of the invention, a compressive force may be applied to the convex surface of a semi elliptical valve head (130, 140) via a compression element (135, 145) so as to bias the flexible material toward the interior of a vacuum chamber but closed at the exterior side of the valve head in order to substantially eliminate variation in opening of the leaflets. The contact surface of the compression element can be flat, raised and or textured as necessary to create the desired bias of the valve head.

In an embodiment of the pressure relief valve the pre-loading of the semi-elliptical valve head assists in the alleviation of the characteristic of the silicone rubber flaps to re-seal and stick together thereby increasing the initial cracking pressure. This pre-loading, shown in Figs. 5 and 6, ensures that the pressure relief device achieves a somewhat consistent and smooth initial opening and approximately consistent subsequent operating cracking pressure regardless of storage times or intervals between usage.

By way of example, FIG. 10 shows a graphical representation of a series of three consecutive flow cycles using a semi-elliptical pressure relief valve that is not compressed in this manner. For each cycle, as vacuum begins to increase 325 the vacuum increases until it reaches the cracking pressure of the valve. In the trace corresponding to the first cycle of the valve 360, it can be observed that this first cracking pressure 375 is substantially lower than then that seen in the second 365 and third 370 traces (corresponding to the second and third flow-cycles of the pressure relief valve). Of note, during the first cycle and following the first cracking pressure 375 it is further observed that the pressure relief valve operates in a varied and erratic manner as a result of the higher pressure required to crack/open the semi-elliptical pressure relief valve. It is of further note that the cracking pressure of the second and third cycles are virtually indistinguishable 380 and the traces following the cracking pressure are smooth indicating smooth operation of the valve. When the flow is terminated, the decay in pressure can be observed 345 until the valve closes 350.

In a preferred embodiment, a semi-elliptical pressure relief valve is compressed (or "pre-loaded") (as illustrated in FIG. 5) in order to eliminate the variances seen in cracking pressure (e.g., shown in. FIG. 10, 375). FIG. 11 shows a graphical representation of a series of consecutive flow cycles using a pre-loaded semi-elliptical pressure relief valve. For each cycle, as vacuum levels begin to increase 325 the vacuum increases until it reaches the cracking pressure 390 of the pressure relief valve. In the traces corresponding to the first and subsequent cycles of the valve, it can be observed that this each cracking pressure 390 is almost identical throughout.

In certain embodiments of the invention, the pressure relief device may contain more than one pressure relief valve element. In the event the negative pressure exceeds both first and second pressure relief valve element tolerances both valves will open, in series, allowing flow 210 into the device. Each pressure relief valve element will remain open until such time as the pressure returns to a value below the operating pressure of second pressure relief valve and subsequently the first pressure relief valve. FIG 12 is a graphical representation showing several flow cycles and corresponding (overlapping) traces of a pressure relief device comprising two pre-compressed pressure relief valve elements (as seen in FIG 3 and FIG 4). Wherein vacuum is applied through a vacuum aperture 110 and critical orifice 120 and air flows out of the device 200 creating a negative pressure within the inner chamber volume 105 and chamber volume 100. The lower line 385 indicates the opening pressure value of a second pressure relief valve, the central line 310 indicating the opening pressure of a first pressure relief valve and the upper line 340 indicating the pressure supplied by the flow from an external regulated supply..

In the graph of FIG. 12, the upper trace 300 shows the flow (in liters per minute as a function of time) through the relief valve; and the lower trace 305 shows vacuum level as a function of time. In this example, both first 103 and second 113 pressure relief valves elements are open in response to the cracking pressure 330 allowing flow 210 into the device until the pressure in the device increases enough to allow for the closure of the second pressure relief valve 113, at which point flow is only through the first pressure relief valve element 103.

In embodiments of the pressure relief valve comprising the silicone rubber semi-elliptical valve head, the deflection of the leaflets can be changed, increased or decreased by modifying physical characteristics of the valve in order to maintain the desired actuating therapeutic pressure range(s). As used herein the deflection is the degree to which the pressure relief valve is displaced by the load generated by the negative pressure within in the device.

By way of example, a silicone rubber semi-elliptical pressure relief valve may contain two slits, perpendicular to one another that extend radially from the center of the silicone rubber valve dome. In one example of the device the semi-elliptical valve head is approximately about 28 mm in height with an approximate diameter of about 6 mm and the compression by the compression element is between about 0.25 mm - 0.8 mm to achieve a consistent cracking pressure that is about 48 hPa and where the natural control range of re-sealing pressure of the relief valve is about 32 hPa.

Hysteretic control may be particularly useful for providing a range of operating pressures and the time to reach said pressures that eliminate a pressure relief valve from abruptly opening and closing (fluttering) caused by the values of the open and closed states being and the time to reach said states being too short. In embodiments of the invention, the hysteretic control range is between about 5 hPa and about 15 hPa, 5 hPa and 10 hPa or less of the operating pressure of the device. In embodiments of the invention where the pressure relief device comprises more than one pressure relief valve element, the hysteretic control range of the pressure relief valve elements may overlap to further enhance smooth transition between a first predetermined pressure control value and second predetermined pressure control value and so on.

Those skilled in the art will appreciate that the conception upon which this disclosure is based may readily be utilized as a basis for the designing of other structures, methods and systems for carrying out the several purposes of the present disclosure. The scope of the invention is defined by the appended claims.

Structural embodiments of the apparatus may vary based on the size of the device and the description provided herein is a guide to the functional aspects and means.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention, which is defined by the appended claims.

All patents and publications mentioned in the specification are indicative of the levels of those of ordinary skill in the art to which the invention pertains.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

## Claims

1. A negative pressure therapy device (200, 300, 400), comprising:
a chamber element comprising a housing (100) configured to mate with and cover a portion of an individual and thereby enclose an inner chamber volume (105) over the covered portion of the individual, wherein the housing (100) separates the inner chamber volume (105) from an ambient gaseous atmosphere external to the chamber device,
wherein the housing (100) comprises
a vacuum aperture (110) configured to be mated to a vacuum source (119, 121) for evacuation of a gas from within the inner chamber volume (105) formed upon mating of the chamber element (100) with the individual, and
a valve (103) which opens at a first predetermined level of vacuum within the inner chamber volume (105) to create an opening through the housing (100) through which gas from the ambient gaseous atmosphere can enter the inner chamber volume (105) and which closes at a second predetermined level of vacuum within the inner chamber volume (105), wherein the second predetermined level of vacuum is lower than the first predetermined level of vacuum,
**characterized in that** the valve (103) comprises
a semi-elliptical valve head (125, 130, 140) having a marginal edge (165), an interior side (163) facing the inner chamber volume (105), an exterior side facing the ambient gaseous atmosphere,
a resilient sleeve (160) at the marginal edge (165) thereof, and
a valve aperture (170, 175) in the valve head (125, 130, 140) which reversibly opens to permit fluid flow (205) therethrough,
wherein the valve head (125, 130, 140) has a generally convex orientation relative to the housing (100) when the valve (103) is in a closed position, and wherein the resilient sleeve (160) is configured and arranged to double over and extend in a rolling manner to apply a torque to the valve head (125, 130, 140) which causes the valve head (125, 130, 140) to invert to a generally concave orientation that assists in opening the valve aperture (170, 175) at or greater than the first predetermined level of vacuum and to revert to its generally convex orientation at or less than the second predetermined level of vacuum.

2. A negative pressure therapy device according to claim 1, wherein the chamber device comprises a compression element (135, 145) that creates a compressed state by applying a compressive force to the exterior side of the valve head (125, 130, 140) when the pressures in the inner chamber volume (105) and the ambient gaseous atmosphere are equal and the valve (103) is closed, wherein the compressive force is configured and arranged to deflect the exterior side of the valve head inward such that the valve aperture (170, 175) is open at the interior side (163) of the valve head (125, 130, 140) but closed at the exterior side of the valve head (125, 130, 140) in the compressed state.

3. A negative pressure therapy device according to claim 1 or 2, further comprising a vacuum source (119, 121) operably connected to the vacuum aperture (110) and configured to evacuate the gas from the inner chamber volume (105) when energized, wherein optionally the vacuum source (119, 121) provides a gas flow rate at the vacuum source (119, 121) that is sufficient to produce a level of vacuum within the inner chamber volume (105) that is greater than the first predetermined level of vacuum when the valve (103) is open, and wherein optionally a gas flow rate through the vacuum aperture (110) is limited by a cross sectional area of a flow passage positioned between the inner chamber volume (105) and the vacuum source (119, 121) that provides a critical orifice (120) type of gas flow, wherein the gas flow rate is limited by the critical orifice (120) type of gas flow to a rate that maintains a level of vacuum within the inner chamber volume (105) that is between the first predetermined level of vacuum and the second predetermined level of vacuum when the valve (103) is open and optionally the gas flow rate is limited by the critical orifice (120) type of gas flow to a rate of approximately 2 liters per minute.

4. A negative pressure therapy device according to one of claims 1-3, wherein the housing (100) further comprises a second valve (113) which opens at a third predetermined level of vacuum within the inner chamber volume (105) to create a second opening through the housing (100) through which gas from the ambient gaseous atmosphere can enter the inner chamber volume (105), wherein the third predetermined level of vacuum is greater than the first predetermined level of vacuum, wherein optionally the third predetermined level of vacuum is between about 47.1 hPa and 137 hPa.

5. A negative pressure therapy device according to one of claims 1-4, wherein the first predetermined level of vacuum and second predetermined level of vacuum is between about 31.3 hPa and about 47.1 hPa.

6. A negative pressure therapy device according to one of claims 1-5 wherein the device (200, 300, 400) is a medical device.

7. A negative pressure therapy device according to one of claims 1-6, wherein the medical device is a negative pressure chamber configured to cover an external portion of a human body, wherein optionally the negative pressure chamber is a Negative-pressure wound therapy (NPWT) device or the negative pressure chamber is a continuous negative external pressure (cNEP) therapy device adapted for maintenance of airway patency by the application of an external vacuum to a human.

8. A negative pressure therapy device according to any one of claims 1 to 7 for use in a method of treating a disease, wherein the method comprises a method of regulating vacuum levels using the negative pressure therapy device (200, 300, 400) of any one of claims 1 to 7, comprising:
providing a chamber device (200, 300, 400) having a housing (100) that encloses an inner chamber volume (105) comprising a gas to be evacuated from within the chamber device (200, 300, 400) by application of a vacuum, wherein the housing (100) separates the inner chamber volume (105) from an ambient gaseous atmosphere external to the chamber device (200, 300, 400), wherein the housing (100) comprises a valve (103) which opens at a first predetermined level of vacuum within the inner chamber volume (105) to create an opening through the housing (100) through which gas from the ambient gaseous atmosphere can enter the inner chamber volume (105) and which closes at a second predetermined level of vacuum within the inner volume (105), wherein the second predetermined level of vacuum is lower than the first predetermined level of vacuum,
wherein the valve (103) comprises
a semi-elliptical valve head (125, 130, 140) having a marginal edge (165), an interior side (163) facing the inner chamber volume (105), an exterior side facing the ambient gaseous atmosphere,
a resilient sleeve (160) at the marginal edge (165) thereof, and
a valve aperture (170, 175) in the valve head (125, 130, 140) which reversibly opens to permit fluid flow (205) therethrough,
wherein the valve head (125, 130, 140) has a generally convex orientation relative to the housing (100) when the valve (103) is in a closed position, and wherein the resilient sleeve (160) is configured and arranged to double over and extend in a rolling manner to apply a torque to the valve head (125, 130, 140) which causes the valve head (125, 130, 140) to invert to a generally concave orientation that assists in opening the valve aperture (170, 175) at or greater than the first predetermined level of vacuum and to revert to its generally convex orientation at or less than the second predetermined level of vacuum; and
operably connecting the chamber device to a vacuum source (119, 121) to evacuate the gas from the inner volume (105), wherein opening and closing of the valve (103) regulates the level of vacuum within the inner volume (105) to a range between the first predetermined level of vacuum and the second predetermined level of vacuum.

9. The negative pressure therapy device for use of claim 8, wherein the method of regulating vacuum levels further comprises that the housing (100) further comprises a second valve (113) which opens at a third predetermined level of vacuum within the inner chamber volume (105) to create a second opening through the housing (100) through which gas from the ambient gaseous atmosphere can enter the inner chamber volume (105), wherein the third predetermined level of vacuum is greater than the first predetermined level of vacuum , wherein optionally the valve (113) is configured to be selectively and reversibly blocked from permitting flow there through, such that blockage of the valve causes the level of vacuum within the inner chamber volume (105) reaches the third predetermined level of vacuum and the second valve opens until the block is reversed.

10. The negative pressure therapy device for use of any one of claims 8 or 9, wherein the disease comprises sleep apnea, snoring, full or partial upper airway collapse, full or partial upper airway, obstruction, a wound, which is optionally caused by an injury or a surgery.

## Patentansprüche

1. Eine Vorrichtung zur Unterdrucktherapie (200, 300, 400), umfassend:
ein Kammerelement, das ein Gehäuse (100) umfasst, das so konfiguriert ist, dass es an ein Individuum angepasst wird und einen Teil dieses bedeckt, wodurch ein inneres Kammervolumen (105) über dem bedeckten Teil des Individuums umschlossen wird,, wobei das Gehäuse (100) das innere Kammervolumen (105) von einer umgebenden gasförmigen Atmosphäre außerhalb der Kammervorrichtung trennt,
wobei das Gehäuse (100) umfasst
eine Vakuumöffnung (110), die so ausgebildet ist, dass sie mit einer Vakuumquelle (119, 121) verbunden werden kann, um Gas aus dem inneren Kammervolumen (105) abzusaugen, das bei der Verbindung des Kammerelements (100) mit dem Individuum entsteht, und
ein Ventil (103), das sich bei einem ersten vorgegebenen Vakuumniveau innerhalb des inneren Kammervolumens (105) öffnet, um eine Öffnung durch das Gehäuse (100) zu schaffen, durch die Gas aus der gasförmigen Umgebungsatmosphäre in das innere Kammervolumen (105) eintreten kann, und das bei einem zweiten vorbestimmten Vakuumniveau innerhalb des inneren Kammervolumens (105) schließt, wobei das zweite vorbestimmte Vakuumniveau niedriger ist als das erste vorbestimmte Vakuumniveau,
**dadurch gekennzeichnet, dass** das Ventil (103)
einen halbelliptischen Ventilkopf (125, 130, 140) mit einer Randkante (165), einer dem inneren Kammervolumen (105) zugewandten Innenseite (163), einer der umgebenden Gasatmosphäre zugewandten Außenseite,
einer elastischen Hülse (160) an dessen Randkante (165) und
eine Ventilöffnung (170, 175) im Ventilkopf (125, 130, 140), die sich reversibel öffnet, um einen Fluidstrom (205) durch sie hindurch zu ermöglichen, umfasst,
wobei der Ventilkopf (125, 130, 140) eine im Allgemeinen konvexe Ausrichtung relativ zum Gehäuse (100) aufweist, wenn sich das Ventil (103) in einer geschlossenen Position befindet, und wobei die elastische Hülse (160) so konfiguriert und angeordnet ist, dass sie sich umklappt und rollend ausdehnt, um ein Drehmoment auf den Ventilkopf (125, 130, 140) auszuüben, das bewirkt, dass sich der Ventilkopf (125, 130, 140) in eine im Allgemeinen konkave Ausrichtung umkehrt, die das Öffnen der Ventilöffnung (170, 175) bei oder über dem ersten vorbestimmten Vakuumniveau unterstützt, und bei oder unter dem zweiten vorbestimmten Vakuumniveau in seine im Allgemeinen konvexe Ausrichtung zurückkehrt.

2. Vorrichtung zur Unterdrucktherapie nach Anspruch 1, wobei die Kammervorrichtung ein Druckelement (135, 145) umfasst, das einen komprimierten Zustand erzeugt, indem es eine Druckkraft auf die Außenseite des Ventilkopfes (125, 130, 140) ausübt, wenn die Drücke im inneren Kammervolumen (105) und in der umgebenden Gasatmosphäre gleich sind und das Ventil (103) geschlossen ist, wobei die Druckkraft so ausgebildet und angeordnet ist, dass sie die Außenseite des Ventilkopfes nach innen auslenkt, sodass die Ventilöffnung (170, 175) an der Innenseite (163) des Ventilkopfes (125, 130, 140) offen, an der Außenseite des Ventilkopfes (125, 130, 140) jedoch geschlossen ist.

3. Eine Vorrichtung zur Unterdrucktherapie nach Anspruch 1 oder 2, die ferner eine Vakuumquelle (119, 121) umfasst, die funktionsfähig mit der Vakuumöffnung (110) verbunden und so ausgelegt ist, dass sie bei Aktivierung das Gas aus dem inneren Kammervolumen (105) absaugt, wobei die Vakuumquelle (119, 121) optional einen Gasdurchfluss an der Vakuumquelle (119, 121) bereitstellt, die ausreicht, um innerhalb des inneren Kammervolumens (105) ein Vakuumniveau zu erzeugen, das größer ist als das erste vorbestimmte Vakuumniveau, wenn das Ventil (103) geöffnet ist, und wobei optional eine Gasdurchflussrate durch die Vakuumöffnung (110) durch eine Querschnittsfläche eines Strömungskanals begrenzt wird, die zwischen dem inneren Kammervolumen (105) und der Vakuumquelle (119, 121) positioniert wird, der einen Gasstrom vom Typ einer kritischen Öffnung (120) bereitstellt, wobei die Gasdurchflussrate durch die kritische Öffnung (120) auf eine Rate begrenzt wird, die ein Vakuumniveau innerhalb des inneren Kammervolumens (105) aufrechterhält, das zwischen dem ersten vorbestimmten Vakuumniveau und dem zweiten vorbestimmten Vakuumniveau liegt, wenn das Ventil (103) geöffnet ist, und wobei optional die Gasdurchflussrate durch den Gasstrom vom Typ einer kritischen Öffnung (120) auf eine Rate von etwa 2 Litern pro Minute begrenzt wird.

4. Eine Vorrichtung zur Unterdrucktherapie nach einem der Ansprüche 1-3, wobei das Gehäuse (100) ferner ein zweites Ventil (113) umfasst, das sich bei einem dritten vorbestimmte Vakuumniveau innerhalb des inneren Kammervolumens (105) öffnet, um eine zweite Öffnung durch das Gehäuse (100) zu schaffen, durch die Gas aus der gasförmigen Umgebungsatmosphäre in das inneren Kammervolumen (105) eintreten kann, wobei das dritte vorbestimmten Vakuumniveau größer ist als das erste vorbestimmten Vakuumniveau, wobei das dritte vorbestimmten Vakuumniveau optional zwischen etwa 47,1 hPa und 137 hPa liegt.

5. Eine Vorrichtung zur Unterdrucktherapie nach einem der Ansprüche 1-4, wobei das erste vorbestimmte Vakuumniveau und das zweite vorbestimmte Vakuumniveau zwischen etwa 31,3 hPa und etwa 47,1 hPa liegen.

6. Eine Vorrichtung zur Unterdrucktherapie nach einem der Ansprüche 1-5, wobei das Gerät (200, 300, 400) ein medizinisches Gerät ist.

7. Vorrichtung zur Unterdrucktherapie nach einem der Ansprüche 1-6, wobei es sich bei der medizinischen Vorrichtung um eine Unterdruckkammer handelt, die so ausgelegt ist, dass sie einen äußeren Bereich des menschlichen Körpers bedeckt, wobei die Unterdruckkammer wahlweise eine Vorrichtung zur Unterdruck-Wundtherapie (NPWT) ist oder eine Vorrichtung zur Therapie mit kontinuierlichem externem Unterdruck (cNEP), die dazu ausgelegt ist, die Durchgängigkeit der Atemwege durch Anlegen eines externen Unterdrucks an den Menschen aufrechtzuerhalten.

8. Eine Vorrichtung zur Unterdrucktherapie nach einem der Ansprüche 1 bis 7 zur Verwendung bei einem Verfahren zur Behandlung einer Erkrankung, wobei das Verfahren ein Verfahren zur Regulierung von Unterdruckwerten unter Verwendung der Vorrichtung zur Unterdrucktherapie (200, 300, 400) gemäß einem der Ansprüche 1 bis 7 umfasst, das Folgendes umfasst:
Bereitstellen einer Kammervorrichtung (200, 300, 400) mit einem Gehäuse (100), das ein inneres Kammervolumen (105) umschließt, das ein Gas enthält, das durch Anlegen eines Vakuums aus dem Inneren der Kammervorrichtung (200, 300, 400) evakuiert werden soll, wobei das Gehäuse (100) das innere Kammervolumen (105) von einer gasförmigen Umgebungsatmosphäre außerhalb der Kammervorrichtung (200, 300, 400) trennt, wobei das Gehäuse (100) ein Ventil (103) umfasst, das sich bei einem ersten vorbestimmten Vakuumniveau innerhalb des inneren Kammervolumens (105) öffnet, um eine Öffnung durch das Gehäuse (100) zu schaffen, durch die Gas aus der umgebenden gasförmigen Atmosphäre in das innere Kammervolumen (105) eintreten kann, und das bei einem zweiten vorbestimmten Vakuumniveau innerhalb des innere Kammervolumens (105) schließt, wobei das zweite vorbestimmte Vakuumniveau niedriger ist als das erste vorbestimmte Vakuumniveau,
wobei das Ventil (103)
einen halbelliptischen Ventilkopf (125, 130, 140) mit einer Randkante (165), einer dem inneren Kammervolumen (105) zugewandten Innenseite (163), einer der umgebenden Gasatmosphäre zugewandten Außenseite,
einer elastischen Hülse (160) an dessen Randkante (165) und
eine Ventilöffnung (170, 175) im Ventilkopf (125, 130, 140), die sich reversibel öffnet, um einen Fluidstrom (205) durch sie hindurch zu ermöglichen, umfasst,
wobei der Ventilkopf (125, 130, 140) eine im Allgemeinen konvexe Ausrichtung relativ zum Gehäuse (100) aufweist, wenn sich das Ventil (103) in einer geschlossenen Position befindet, und wobei die elastische Hülse (160) so konfiguriert und angeordnet ist, dass sie sich umklappt und rollend ausdehnt, um ein Drehmoment auf den Ventilkopf (125, 130, 140) auszuüben, das bewirkt, dass sich der Ventilkopf (125, 130, 140) in eine im Allgemeinen konkave Ausrichtung umkehrt, die das Öffnen der Ventilöffnung (170, 175) bei oder über dem ersten vorbestimmten Vakuumniveau unterstützt, und bei oder unter dem zweiten vorbestimmten Vakuumniveau in seine im Allgemeinen konvexe Ausrichtung zurückkehrt; und
die Kammervorrichtung funktionsfähig mit einer Vakuumquelle (119, 121) zu verbinden, um das Gas aus dem Innenraum (105) zu entfernen, wobei das Öffnen und Schließen des Ventils (103) das Vakuumniveau innerhalb des Innenraums (105) auf einen Bereich zwischen dem ersten vorbestimmten Vakuumniveau und dem zweiten vorbestimmten Vakuumniveau regelt.

9. Die Vorrichtung zur Unterdrucktherapie zur Verwendung nach Anspruch 8, wobei das Verfahren zur Regelung des Unterdruckniveaus ferner umfasst, dass das Gehäuse (100) ein zweites Ventil (113) umfasst, das sich bei einem dritten vorbestimmten Vakuumniveau innerhalb des inneren Kammervolumens (105) öffnet, um eine zweite Öffnung durch das Gehäuse (100) zu schaffen, durch die Gas aus der gasförmigen Umgebungsatmosphäre in das innere Kammervolumen (105) eintreten kann, wobei das dritte vorbestimmte Vakuumniveau größer ist als das erste vorbestimmte Vakuumniveau, wobei optional das Ventil (113) so konfiguriert ist, dass es selektiv und reversibel daran gehindert werden kann, einen Durchfluss zuzulassen, sodass die Blockierung des Ventils bewirkt, dass das Vakuumniveau innerhalb des inneren Kammervolumens (105) das dritte vorbestimmte Vakuumniveau erreicht und das zweite Ventil geöffnet bleibt, bis die Blockierung aufgehoben wird.

10. Die Vorrichtung zur Unterdrucktherapie zur Verwendung nach einem der Ansprüche 8 oder 9, wobei die Erkrankung Schlafapnoe, Schnarchen, einen vollständigen oder teilweisen Kollaps der oberen Atemwege, eine vollständige oder teilweise Obstruktion der oberen Atemwege oder eine Wunde umfasst, die gegebenenfalls durch eine Verletzung oder einen chirurgischen Eingriff verursacht wurde.

## Revendications

1. Un dispositif de thérapie par pression négative (200, 300, 400), comprenant :
un élément de chambre comprenant un boîtier (100) configuré pour s'adapter à une personne et recouvrir une partie de celle-ci, et ainsi délimiter un volume de chambre interne (105) au-dessus de la partie recouverte de la personne, dans lequel le boîtier (100) sépare le volume de chambre interne (105) d'une atmosphère gazeuse ambiante externe au dispositif de chambre,
dans lequel le boîtier (100) comprend une ouverture de vide (110) configurée pour être raccordée à une source de vide (119, 121) afin d'évacuer un gaz de l'intérieur du volume de chambre interne (105) formé lors de l'accouplement de l'élément de chambre (100) avec l'individu, et
une valve (103) qui s'ouvre à un premier niveau de vide prédéterminé à l'intérieur du volume de chambre interne (105) pour créer une ouverture à travers le boîtier (100) par laquelle le gaz de l'atmosphère gazeuse ambiante peut pénétrer dans le volume de la chambre interne (105) et qui se ferme à un deuxième niveau de vide prédéterminé à l'intérieur du volume de la chambre interne (105), le deuxième niveau de vide prédéterminé étant inférieur au premier niveau de vide prédéterminé,
**caractérisé en ce que** la valve (103) comprend
une tête de soupape semi-elliptique (125, 130, 140) comportant un bord périphérique (165), une face intérieure (163) tournée vers le volume de la chambre interne (105), une face extérieure tournée vers l'atmosphère gazeuse ambiante,
un manchon élastique (160) au niveau de son bord périphérique (165), et
une ouverture de soupape (170, 175) dans la tête de soupape (125, 130, 140) qui s'ouvre de manière réversible pour permettre l'écoulement de fluide (205) à travers celle-ci,
dans laquelle la tête de soupape (125, 130, 140) présente une orientation généralement convexe par rapport au boîtier (100) lorsque la valve (103) est en position fermée, et dans laquelle le manchon élastique (160) est configuré et agencé pour se replier sur lui-même et s'étendre de manière roulante afin d'appliquer un couple à la tête de valve (125, 130, 140) qui provoque l'inversion de la tête de valve (125, 130, 140) de s'inverser pour prendre une orientation généralement concave qui facilite l'ouverture de l'orifice de la soupape (170, 175) à un niveau de vide égal ou supérieur au premier niveau prédéterminé, et de revenir à son orientation généralement convexe à un niveau de vide égal ou inférieur au deuxième niveau prédéterminé.

2. Un dispositif de thérapie par pression négative selon la revendication 1, dans lequel le dispositif à chambre comprend un élément de compression (135, 145) qui crée un état de compression en appliquant une force de compression sur la face extérieure de la tête de soupape (125, 130, 140) lorsque les pressions dans le volume interne de la chambre (105) et dans l'atmosphère gazeuse ambiante sont égales et que la valve (103) est fermée, dans lequel la force de compression est configurée et agencée pour dévier la face extérieure de la tête de valve vers l'intérieur de telle sorte que l'ouverture de la valve (170, 175) soit ouverte du côté intérieur (163) de la tête de valve (125, 130, 140) mais fermée du côté extérieur de la tête de soupape (125, 130, 140) à l'état comprimé.

3. Un dispositif de thérapie par pression négative selon la revendication 1 ou 2, comprenant en outre une source de vide (119, 121) reliée de manière fonctionnelle à l'orifice de vide (110) et configurée pour évacuer le gaz du volume de la chambre interne (105) lorsqu'elle est activée, dans lequel, de manière facultative, la source de vide (119, 121) fournit un débit de gaz au niveau de la source de vide (119, 121) qui est suffisant pour produire un niveau de vide à l'intérieur du volume de la chambre interne (105) supérieur au premier niveau de vide prédéterminé lorsque la vanne (103) est ouverte, et dans lequel, en option, un débit de gaz à travers l'orifice de vide (110) est limité par une section transversale d'un passage d'écoulement situé entre le volume de la chambre interne (105) et la source de vide (119, 121) qui fournit un écoulement de gaz de type à orifice critique (120), dans lequel le débit de gaz est limité par l'orifice critique (120) à un débit qui maintient un niveau de vide à l'intérieur du volume de la chambre interne (105) compris entre le premier niveau de vide prédéterminé et le deuxième niveau de vide prédéterminé lorsque la vanne (103) est ouverte et, en option, le débit de gaz est limité par l'écoulement de type orifice critique (120) à un débit d'environ 2 litres par minute.

4. Un dispositif de thérapie par pression négative selon l'une des revendications 1 à 3, dans lequel le boîtier (100) comprend en outre une deuxième soupape (113) qui s'ouvre à un troisième niveau de vide prédéterminé à l'intérieur du volume de la chambre interne (105) afin de créer une deuxième ouverture à travers le boîtier (100) à travers laquelle du gaz provenant de l'atmosphère gazeuse ambiante peut pénétrer dans le volume de la chambre interne (105), dans lequel le troisième niveau de vide prédéterminé est supérieur au premier niveau de vide prédéterminé, dans lequel, en option, le troisième niveau de vide prédéterminé est compris entre environ 47,1 hPa et 137 hPa.

5. Un dispositif de thérapie par pression négative selon l'une des revendications 1 à 4, dans lequel le premier niveau de vide prédéterminé et le deuxième niveau de vide prédéterminé sont compris entre environ 31,3 hPa et environ 47,1 hPa.

6. Un dispositif de thérapie par pression négative selon l'une des revendications 1 à 5, dans lequel le dispositif (200, 300, 400) est un dispositif médical.

7. Un dispositif de thérapie par pression négative selon l'une des revendications 1 à 6, dans lequel le dispositif médical est une chambre à pression négative conçue pour recouvrir une partie externe du corps humain, dans lequel, éventuellement, la chambre à pression négative est un dispositif de traitement des plaies par pression négative (NPWT) ou la chambre à pression négative est un dispositif de thérapie par pression négative externe continue (cNEP) adapté au maintien de la perméabilité des voies respiratoires par l'application d'un vide externe à un être humain.

8. Un dispositif de thérapie par pression négative selon l'une quelconque des revendications 1 à 7, destiné à être utilisé dans un procédé de traitement d'une maladie, dans lequel le procédé comprend un procédé de régulation des niveaux de vide à l'aide du dispositif de thérapie par pression négative (200, 300, 400) selon l'une quelconque des revendications 1 à 7, comprenant :
la mise à disposition d'un dispositif à chambre (200, 300, 400) comportant un boîtier (100) qui renferme un volume de chambre interne (105) contenant un gaz devant être évacué de l'intérieur du dispositif à chambre (200, 300, 400) par application d'un vide, dans lequel le boîtier (100) sépare le volume de chambre interne (105) d'une atmosphère gazeuse ambiante externe au dispositif à chambre (200, 300, 400), dans lequel le boîtier (100) comprend une vanne (103) qui s'ouvre à un premier niveau de vide prédéterminé à l'intérieur du volume de chambre interne (105) pour créer une ouverture à travers le boîtier (100) par laquelle le gaz de l'atmosphère gazeuse ambiante peut pénétrer dans le volume de la chambre interne (105) et qui se ferme à un deuxième niveau de vide prédéterminé à l'intérieur du volume interne (105), dans lequel le deuxième niveau de vide prédéterminé est inférieur au premier niveau de vide prédéterminé,
dans lequel la soupape (103) comprend
une tête de soupape semi-elliptique (125, 130, 140) comportant un bord périphérique (165), une face intérieure (163) tournée vers le volume de la chambre interne (105), une face extérieure tournée vers l'atmosphère gazeuse ambiante,
un manchon élastique (160) au niveau de son bord périphérique (165), et
une ouverture de soupape (170, 175) dans la tête de soupape (125, 130, 140) qui s'ouvre de manière réversible pour permettre l'écoulement de fluide (205) à travers celle-ci,
dans laquelle la tête de soupape (125, 130, 140) présente une orientation généralement convexe par rapport au boîtier (100) lorsque la valve (103) est en position fermée, et dans laquelle le manchon élastique (160) est configuré et agencé pour se replier sur lui-même et s'étendre de manière roulante afin d'appliquer un couple à la tête de valve (125, 130, 140) qui provoque l'inversion de la tête de valve (125, 130, 140) de s'inverser pour adopter une orientation généralement concave qui facilite l'ouverture de l'orifice de la soupape (170, 175) à un niveau de vide égal ou supérieur au premier niveau prédéterminé, et de revenir à son orientation généralement convexe à un niveau de vide égal ou inférieur au deuxième niveau prédéterminé ; et
raccorder de manière fonctionnelle le dispositif à chambre à une source de vide (119, 121) pour évacuer le gaz du volume intérieur (105), l'ouverture et la fermeture de la soupape (103) régulant le niveau de vide à l'intérieur du volume intérieur (105) dans une plage comprise entre le premier niveau de vide prédéterminé et le deuxième niveau de vide prédéterminé.

9. Le dispositif de thérapie par pression négative selon la revendication 8, dans lequel le procédé de régulation des niveaux de vide comprend en outre le fait que le boîtier (100) comprend en outre une deuxième soupape (113) qui s'ouvre à un troisième niveau de vide prédéterminé à l'intérieur du volume de la chambre interne (105) pour créer une deuxième ouverture à travers le boîtier (100) par laquelle le gaz provenant de l'atmosphère gazeuse ambiante peut pénétrer dans le volume de la chambre interne (105), dans lequel le troisième niveau de vide prédéterminé est supérieur au premier niveau de vide prédéterminé, dans lequel, en option, la valve (113) est configurée pour être bloquée de manière sélective et réversible afin d'empêcher tout écoulement à travers celle-ci, de telle sorte que le blocage de la soupape provoque l'atteinte du troisième niveau de vide prédéterminé à l'intérieur du volume de la chambre interne (105) et que la deuxième soupape reste ouverte jusqu'à ce que le blocage soit levé.

10. Le dispositif de thérapie par pression négative destiné à être utilisé selon l'une quelconque des revendications 8 ou 9, dans lequel l'affection comprend l'apnée du sommeil, le ronflement, l'affaissement total ou partiel des voies respiratoires supérieures, l'obstruction totale ou partielle des voies respiratoires supérieures, ou une plaie, qui peut être causée par une blessure ou une intervention chirurgicale.
